# FASCICULE DE BREVET EUROPEEN

(11) **EP 2 854 673 B1**
(45) Date de publication et mention de la délivrance du brevet: **07.03.2018**
(21) Numéro de dépôt: 13729998.8
(22) Date de dépôt: 28.05.2013
(51) Int. Cl.: A61B 17/70, A61B 17/02

(54) **DISPOSITIF RACHIDIEN COMPRENANT DES MOYENS D'ACCROCHE RÉVERSIBLE**
WIRBELSÄULENVORRICHTUNG MIT MITTEL ZUR REVERSIBLEN BEFESTIGUNG
SPINAL DEVICE COMPRISING MEANS OF REVERSIBLE FASTENING

(30) Priorité: 28.05.2012 FR 1254895
(43) Date de publication de la demande: 08.04.2015
(73) Titulaire: Safe Orthopaedics, 95610 Eragny-sur-Oise (FR)
(72) Inventeur: PETIT, Dominique, F-62180 Verton (FR)
(74) Mandataire: IP Trust
(86) Numéro de dépôt international: PCT/FR2013/051191
(87) Numéro de publication internationale: WO 2013/178940

(56) Documents cités:
- FR-A1- 2 954 689
- US-A1- 2006 264 962
- US-A1- 2008 262 318
- US-A1- 2010 198 268

## Description

### Domaine de l'invention

La présente invention concerne le domaine des instruments chirurgicaux pour les interventions de stabilisation rachidienne par élément d'ancrage osseux de type vis par voies postérieure ou postéro-latérale.

L'invention concerne plus particulièrement un kit d'instrumentation selon l'invention destiné notamment, mais non exclusivement, à la chirurgie d'ostéosynthèse rachidienne lombaire, thoracique, ou encore cervicale postérieure par voies chirurgicale minimalement invasive ou ouverte.

Lors de dysfonctionnements anatomiques de la colonne vertébrale, on procède à la mise en place d'ancrages osseux de type vis pédiculaire ou vertébrales dans les vertèbres reliées entre elles par des éléments de liaison de type tiges ou plaques.

### Etat de la technique

On connaît dans l'état de la technique la demande de brevet PCT/FR10/000880 (WO2011080426) de la demanderesse. Ce document divulgue une instrumentation pour la fixation d'au moins deux vertèbres rachidiennes par implants d'ancrage osseux de type vis pédiculaires comprenant un élément d'ancrage osseux destinés à être fixé sur une vertèbre, pré-monté sur un tube de montage à usage unique, et un emballage scellé de conditionnement stérile.

Ce document de l'art antérieur concerne également un kit d'instruments pour la pose ou la dépose d'un implant rachidien comportant au moins deux éléments d'ancrage osseux filetés, un organe de liaison de type tige ou plaque reliant mécaniquement les éléments d'ancrage osseux et des éléments de blocage pour verrouiller en position l'élément de liaison par rapport aux éléments d'ancrage, pour réaliser la totalité des gestes chirurgicaux liés à la pose ou la dépose dudit implant, caractérisé en ce que la totalité de ces instruments nécessaires sont à usage unique, conditionnés stérile dans un ou plusieurs emballages scellés.

On connaît également le brevet français FR2874496 décrivant un rétracteur de tissus d'un patient, du type comprenant deux lames présentant respectivement une extrémité proximale et une extrémité distale, lesdites lames étant disposées pour former un canal opératoire ouvert aux extrémités proximales et aux extrémités distales desdites lames, caractérisé en ce que le rétracteur comporte au moins une lame complémentaire pour former un rétracteur à trois lames au moins, lesdites lames s'écartant les unes des autres par pivotement de leurs extrémités distales de sorte à former un canal opératoire élargi de forme conique.

On connaît aussi la demande de brevet européen EP0455282 décrivant un écarteur auto-statique comprenant un châssis d'enrouleur polygonale associé à une pluralité de dilatateurs.

Lorsqu'il est inséré profondément dans le corps du patient il maintient les bords de l'incision ouverts.Les côtés sont reliés de manière articulée les uns aux autres et deux charnières opposées.

Un autre exemple d'écarteur est décrit dans le brevet américain US3509873. Le document US 2006/0264962 décrit un système de fusion de vertèbres postérieures pouvant comprendre une pluralité de canules, chaque canule comportant deux lames qui peuvent être maintenues parallèles entre elle à l'aide d'un élément d'appui.

### Inconvénients de l'état de la technique

Ces solutions de rétracteurs de l'art antérieurprésentent deux inconvénients majeurs.En premier lieu, ces solutions se traduisent par deux dispositifs distincts :
- un ou plusieurs tubes permettant de maintenir la vis rachidienne et de guider au cours de l'intervention la tige de liaison qui vient s'insérer dans la tête de la vis, puis d'engager et visser un bouchon verrouillant la tige dans la tête de la vis
- un deuxième dispositif constitué par plusieurs lames apte à maintenir les tissus aux abords de la zone d'intervention.

Dans les solutions de l'art antérieur, ces dispositifs connus occupent un volume important dans la zone chirurgical, ce qui oblige soit à élargir l'incision, soit à accepter un champ de vision et de travail étroit. Dans les deux cas, le chirurgien est gêné dans l'exécution de l'intervention et du geste chirurgical.

Le deuxième inconvénient est que le deuxième dispositif doit être positionné avant l'insertion des vis. Une fois qu'il est placé dans la zone d'intervention, il vient limiter l'angulation possible des tubes et rend donc plus difficile la visée rachidienne et l'engagement de la zone proximale du tube sur la vis rachidienne lorsque la vis n'est pas pré-montée sur le tube.

Enfin, dans toutes les solutions de l'art antérieur, le rétracteur (deuxième dispositif) est constitué par un instrument chirurgical complexe, nécessitant une stérilisation minutieuse et difficile, en raison des formes complexes et de la présence de multiples articulations, après chaque utilisation. Ce dispositif supplémentaire complexe occasionne par ailleurs un surcoût difficilement supportable au regard des contraintes d'économie de la santé.

### Solution apportée par l'invention

La présente invention est définie par la revendication indépendante 1 tandis que des modes de réalisations préférées sont exposés dans les revendications dépendantes. Afin de remédier aux inconvénients de l'art antérieur, la présente invention propose une solution consistant à compléter le ou les tube(s) rachidien(s) par un accessoire simple au moins permettant d'utiliser le tube non seulement pour sa fonction initiale de pose la vis rachidienne, d'insertion de la tige de liaison et de serrage du bouchon, mais aussi de conférer au(x) tube(s) une fonction annexe de rétractation des tissus.

A cet effet, l'invention concerne selon son acception la plus générale un dispositif rachidien pour la réalisation d'une intervention chirurgicale sur des vertèbres par voie postérieure ou postéro-latérale, caractérisé en ce qu'il comprend au moins un tube, au moins une vis rachidienne apte à coopérer avec l'extrémité proximale d'un tube, caractérisé en ce que le dispositif comportant en outre un accessoire de liaison, présentant des moyens d'accrochage réversible sur l'un au moins desdits tubes, ladite pièce de liaison étant configurée pour maintenir au moins l'un desditstubes dans une orientation angulaireouverte.

On entend par « orientation angulaire ouverte » une orientation dans laquelle une génératrice du tube est divergente, du coté distale, avec l'axe longitudinal de la vis rachidienne.

Avantageusement, comprend deux tubes forméschacun de deux demi-coques aptes à coopérer avec la tête d'une vis rachidienne d'une manière permettant un basculement proximal de l'une au moins desdites demi-coques par rapport à la tête de ladite vis rachidienne, ledit accessoire de liaison étant configuré pour maintenir deux desdites demi-coques avec une orientation angulaire ouverte.

Selon une première variante, ledit accessoire de liaison est constitué par une pièce adaptable auxdits deux demi-coques d'un même tube, ladite pièce présentant une partie médiane prolongée de part et d'autre par une zone d'accrochage réversible apte à coopérer avec une zone distalecomplémentaire d'une desdites demi-coque, afin de verrouiller lesdites demi-coques en position ouverte.

Avantageusement,ladite pièce de liaison présente deux zones opposées d'accrochage réversible sur une zone frontale d'une desdites demi-coque, lesdites zones présentant une surface intérieure complémentaire de la surface extérieure de la zone frontale des demi-coques.

Selon une deuxième variante, non exclusive de la première variante,ledit accessoire de liaison est constitué par une lame de rétraction rigide apte à coopérer avec deux tubes distincts et à maintenir écartés les tissus de l'abord chirurgical par un verrouillage de l'orientation relative de deux tubes.

Selon un mode de réalisation particulier, le dispositif comprend deux tubes constitués chacun de deux demi-coques, de deux vis rachidiennes et deux accessoires de liaison.

L'invention concerne également un accessoire de liaison de deux demi-coques, caractérisé en ce qu'il est constitué par une pièce rigide présentant des moyens d'accrochage réversible sur lesdites demi-coques, ladite pièce de liaison étant configurée pour maintenir lesdites demi-coques dans une position ouverte.

L'invention concerne encore une lame de rétraction pour la réalisation d'une intervention chirurgicale sur des vertèbres par voie postérieure ou postéro-latérale, caractérisé en ce qu'elle est constituée par une lame rigide apte à coopérer avec deux tubes distincts et à maintenir écartés les tissus de l'abord chirurgical.

Un tel accessoire de liaison et une telle lame de rétractation sont les composants d'un dispositif conforme à l'invention. Ils peuvent être utilisés conjointement ou séparément pour la mise en oeuvre de l'invention consistant à conférer à des tubes rachidiens une fonction additionnelle de rétractation des tissus entourant la zone d'intervention chirurgicale en limitant l'encombrement de l'instrumentation afin de préserver pour le chirurgien la meilleure vision sur la zone d'intervention.

Selon une variante avantageuse, l'accessoire de liaison est constitué par une lame présentant une zone médiane prolongée de part et d'autre par des gouttières autorisant un déplacement relatif longitudinal des tubes logés dans lesdites gouttières.

### Description détaillée d'exemples non limitatifs de réalisation

D'autres objets et avantages de l'invention apparaîtront au cours de la description qui suit, faite en référence aux dessins annexés, dans lesquels :
- la figure 1 représenteune vue en perspective de deux tubes lors de mise en place des vis dans une vertèbre
- la figure 2 représente une vue d'une première variante de réalisation, en perspective, de deux tubes verrouillés en position ouverte par un premier accessoire de liaison
- la figure 3 représente une vue de profil de la réalisation selon ladite première variante, d'un tube en position verrouillée ouverte
- la figure 4 représente une vue en coupe, dudit premier accessoire de liaison
- la figure 5 représente une vue selon un plan de coupe sagittal d'une deuxième variante de réalisation des deux tubes reliés par un second accessoire de liaison
- la figure 6 représente une vue selon un premier exemple de réalisation dudit deuxième accessoire de liaison, en vue de profil
- la figure 7 représente une vue selon un deuxième exemple de réalisation dudit deuxième accessoire de liaison, en vue de profil
- les figures 8 à 10 représententdes vues respectivement de dessus, de profil et de coté selon un troisième exemple de réalisation dudit deuxième accessoire de liaison,
- la figure 11 représente une vue de dessus d'un mode de réalisation combinant lesdits premier et deuxième accessoire de liaison,
- la figure 12 représente une vue en perspective d'une troisième variante de réalisation des deux tubes reliés par un troisième accessoire de liaison,
- la figure 13 représente une vue de dessus de la réalisation de la troisième variante illustrée sur la figure 12,
- la figure 14 représente une vue en perspective du troisième accessoire de liaison.

### Présentation sommaire des tubes connus dans l'art antérieur.

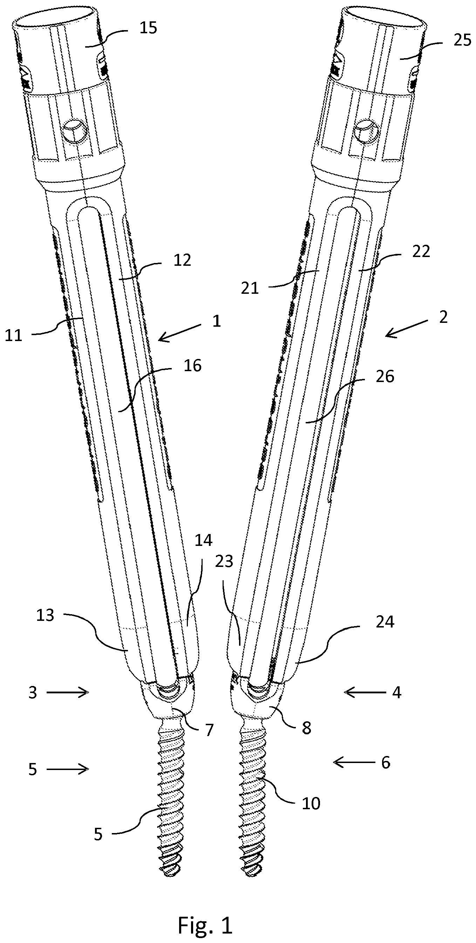

La figure 1 représente une vue de profil de deux tubes (1, 2) lors de mise en place des vis (5, 6) dans une vertèbre, avec des tubes connus dans l'art antérieur. Chacun de ces deux tubes (1, 2) supporte à son extrémité proximale (3, 4) une vis (5, 6) rachidienne (ou « pédiculaire ») qui est avantageusement pré-montée. La liaison entre l'extrémité proximale (3, 4) et la vis (5, 6) est assurée par l'intermédiaire d'une tête (7, 8) présentant une gorge d'accrochage coopérant avec un épaulement prévus à la surface intérieure de l'extrémité proximale (3, 4) des tubes (1, 2).

Les vis pédiculaires (5, 6) sont destinées à être fixée sur des vertèbres. Elles comprennent un moyen d'ancrage osseux (9, 10) prolongé par une tête (7, 8) fendue pour recevoir une tige de liaison intervertébrale non représentée. Lorsque la tige est en place, un bouchon vissable (non représenté) vient se visser dans la tête (7, 8) par un filetage, pour verrouiller l'ensemble.

Le matériau le plus fréquemment utilisé pour la fabrication des vis est le titane. Dans une configuration particulière de l'invention le matériau utilisé pour la fabrication peut être tous matériaux implantables connus ou non à ce jour, comme le Peek, l'inox, le chrome cobalt, ou encore un composite à base de fibre de verre ou de carbone. Des revêtements de type HATCP (HydroxyApatiteTriCalcium Phosphate) ou autres peuvent également être appliqués pour améliorer l'ancrage osseux ou bien la tenue mécanique globale de l'implant.

La pose de cette vis (5, 6) et la mise en place de la tige, puis son verrouillage avec le bouchon vissable sont assurés par un instrument décrit dans le brevet de l'art antérieur PCT/FR10/000880 dont le contenu est incorporé à la présente demande par référence à cette demande PCT.

Les tubes (1, 2) sont, dans l'exemple décrit, chacun constitués de deux demi-coques respectivement (11, 12), (21, 22) articulés à leurs extrémités proximales (13, 14), (23, 24) d'une manière autorisant un basculement par rapport à la tête de la vis (7, 8), lorsque les deux demi-coques sont libérées.

Une bague (15, 25) assure le verrouillage des deux demi-coques (11, 12) pour former des tubes (1, 2) fermés lors de certaines phases d'utilisation.

Les tubes (1, 2) présentent des lumières longitudinales (16, 26) permettant l'engagement d'une tige de liaison et son déplacement jusque dans la fente en « U » prévue dans la tête (7, 8) de la vis.

Il est à noter que l'invention n'est pas limitée à la mise en oeuvre de tubes formés de deux demi-coques, et peut s'appliquer également à des tubes constitués d'une seule pièce.

Toutefois, la réalisation sous forme de deux demi-coques assemblées constitue un mode de réalisation préféré.

### Description détaillée de la première variante de réalisation.

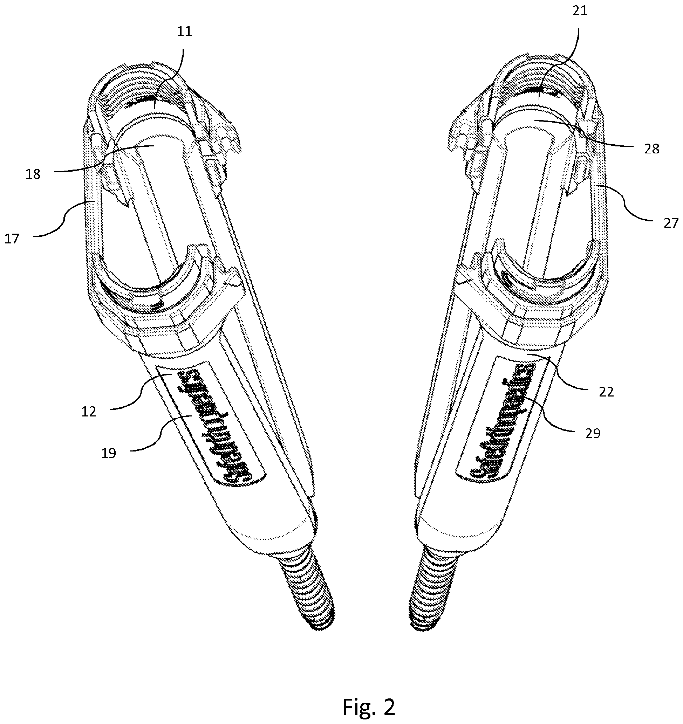

La figure 2 représente une vue d'une première variante de réalisation, en perspective, de deux tubes (1, 2)verrouillés en position ouverte par un premier accessoire de liaison (17, 27).

Les tubes (1, 2) sont dépourvus de la bague (15, 25) afin de permettre l'écartement angulaire des deux demi-coques (11, 12), (21, 22).

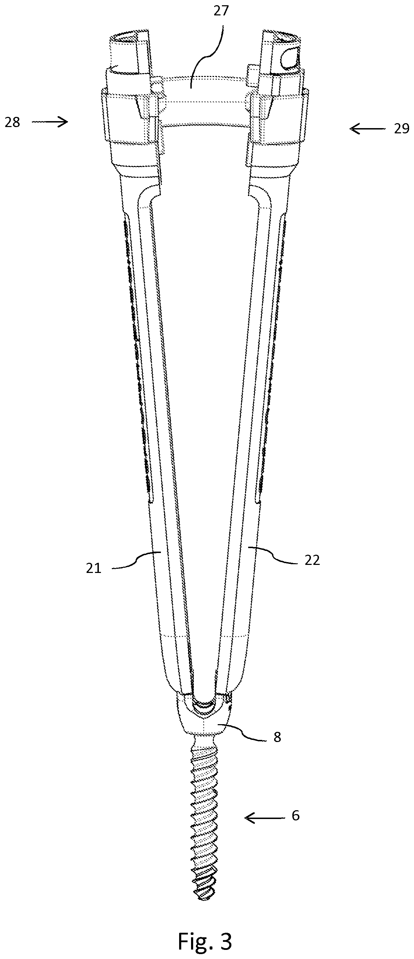

La figure 2 représente une implantation postérieure dorsale par des tubes dont un exemple est illustré en détail par la figure 3. Chaque tube (1, 2) porte une vis implantée dans les pédicules de deux vertèbres distincts, pas nécessairement consécutives. Les deux tubes (1, 2) sont décalés selon un axe sagittal parallèle à la colonne vertébrale. Les demi-coques (11, 12), (21, 22) sont ouvertes pour former chacune un angle par rapport au plan sagittal. Cette ouverture permet de dégager le champ de vision et de travail sur la zone d'implantation de la vis correspondante.

L'écartement des deux demi-coques ne désolidarise pas le tube (1, 2) de la tête (7, 8) de la vis (5, 6) en raison de l'articulation entre ces deux pièces par un épaulement coopérant avec un rainure, comme enseigné par la demande de brevet PCT '880 susvisée.

Le maintien en position ouverte des demi-coques (11, 12), (21, 22) est assuré par une accessoire de liaison (17, 27) qui vient s'adapter sur les extrémités distales (18, 19), (28, 29) des demi-coques (11, 12), (21, 22).

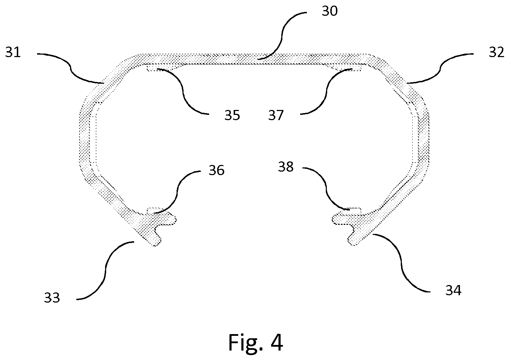

La figure 4 représente une vue détaillée de l'accessoire de liaison.Il est constitué par une pièce moulée en matière plastique dans l'exemple décrit, présentant une forme générale en « C ». La partie médiane (30) présente une longueur définissant l'angle d'ouverture du tube, lorsque l'accessoire est mis en place sur les deux demi-coques.

A titre d'exemple cette partie médiane (30) présente une longueur de 25 millimètres, pour une angulation de 20 degrés entre les deux demi-coques (21, 22), lorsque celle-ci présente une longueur de 150 millimètres, mesuré entre la zone de liaison avec la tête de vis d'une part, et la zone de liaison avec l'accessoire d'autre part.

La partie médiane (30) est prolongée de part et d'autre par une partie arquée (31, 32) dont la section intérieure peut venir épouser la surface extérieure de l'extrémité distale les extrémités distales (18, 19), (28, 29) des demi-coques (11, 12), (21, 22).

L'ouverture de ces parties arquées (31, 32) est déterminée pour permettre un engagement par déformation élastique sur lesdites extrémités distales (18, 19), (28, 29) des demi-coques (11, 12), (21, 22), et assurer un maintien par clipsage.

Chaque partie arquée (31, 32) se prolonge par une patte (33, 34) facilitant le démontage. Ces pattes (33, 34) permettent d'exercer des efforts radiaux afin d'ouvrir la partie arquée, lorsque l'on veut retirer l'accessoire.

La surface intérieure de l'accessoire présente également des ergots (35 à 38) venant se loger dans des cavités complémentaires prévues à la surface intérieure desdites extrémités distales (18, 19), (28, 29) des demi-coques (11, 12), (21, 22), et améliorer le positionnement et maintien de l'accessoire.

Les deux demi-tubes assurent une fonction de rétracteur latéral, lorsqu'ils sont verrouillés en position ouverte par l'accessoire selon le première variante de réalisation, sans qu'il ne soit nécessaire d'introduire dans l'incision un outillage supplémentaire.

### Description détaillée de la deuxième variante de réalisation.

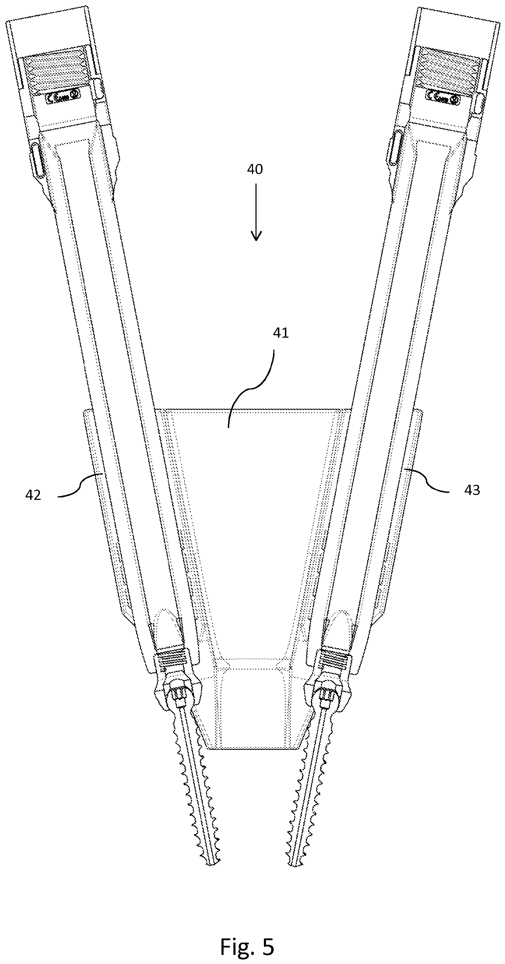

La figure 5 représente une vue selon un plan de coupe sagittal d'une deuxième variante de réalisation, des demi-coques de deux tubes reliés par un second accessoire de liaison.

Cet accessoire ne sert pas à relier les deux demi-coques d'un même tube pour exercer une pression latérale, selon une direction perpendiculaire au plan sagittal, comme prévu pour la première variante.

Il sert à relier deux tubes distincts, qui peuvent être constitué d'une seule pièce ou de deux demi-coques, et de positionner un accessoire formant rétracteur, s'étendant dans une direction longitudinale.

Cet accessoire est constitué par une pièce en matière plastique (40) dans l'exemple décrit. Elle présente une zone médiane (41) présentant la forme d'un segment tronconique mince, pour former un voile bombé, prolongée de part et d'autre par une gouttière (42, 43) de forme complémentaire à la section extérieure desdits tubes.

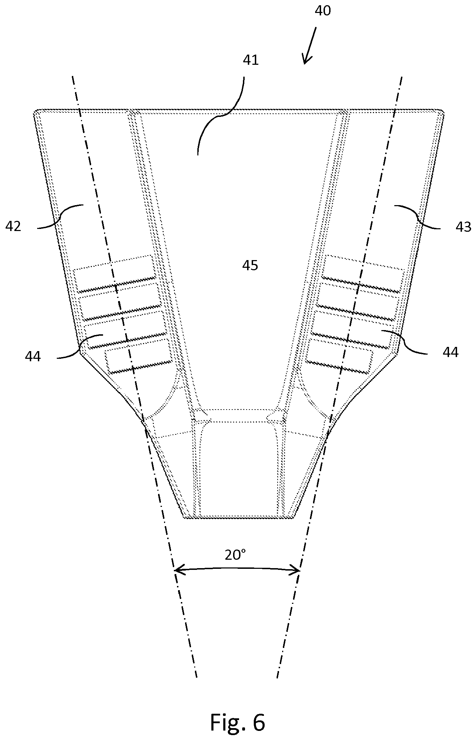

Les deux gouttières (42, 43) présentent des axes convergents en direction proximale, avec un angle d'environ 20 degrés. La surface intérieure de ces gouttières (42, 43) peut présenter des cannelures (44) comme représenté en figure 6 pour assurer le maintien vertical par rapport aux tubes (1, 2). D'autres modes d'accrochages sont envisageables, par exemple un ou plusieurs plots, ergots ou protubérances venant s'enclencher dans un ou plusieurs logement(s) complémentaire(s) prévu(s) à la surface extérieure des tubes (1, 2). Ces plots, ergots ou protubérances sont de préférences configurées afin de permettre un déplacement longitudinal des tubes par rapport aux gouttières, avec des positions indexées par exemple.

Le déplacement longitudinal de l'accessoire par rapport aux tubes provoque une variation de l'écartement des extrémités proximales des tubes (1, 2) et par conséquent exerce une contrainte d'écartement des vis ancrées sur les vertèbres d'une part et les tubes (1, 2) d'autre part. Ceci permet d'améliorer la vision sur la zone d'intervention intervertébrale, facilitant le geste chirurgical, par exemple plus l'insertion d'une cage intersomatique par voie transforaminale. En repoussant l'accessoire dans le sens proximal, on provoque ainsi une ouverture forcée de l'espace intervertébral.

La figure 6 fait également apparaître que la zone centrale (41) présente une première partie distale (45) en forme de tuile tronconique de section décroissante vers la direction proximale, prolongée par une deuxième partie proximale (46) en forme de tuile cylindrique, évasée par rapport à la première partie distale (45). L'axe de symétrie de cette deuxième partie forme un angle divergent avec l'axe de symétrie de la première partie, de façon à ce que la zone distale de l'accessoire repousse les tissus proches des vertèbres vers l'extérieur lorsqu'il est engagé dans la zone d'intervention chirurgicale.

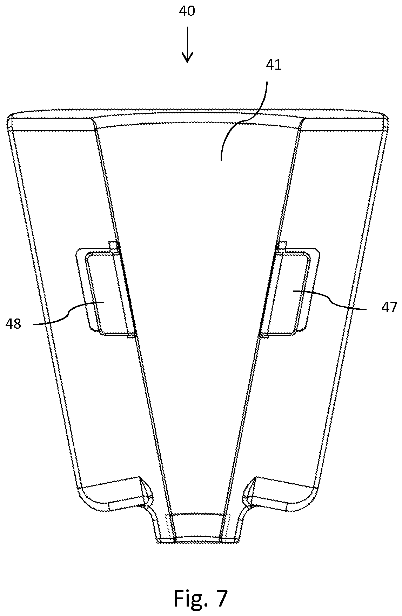

Les gouttières peuvent être remplacées par des ergots (47, 48) assurant la même fonction d'ancrage de l'accessoire sur les tubes (1, 2) comme représenté sur la figure 7.

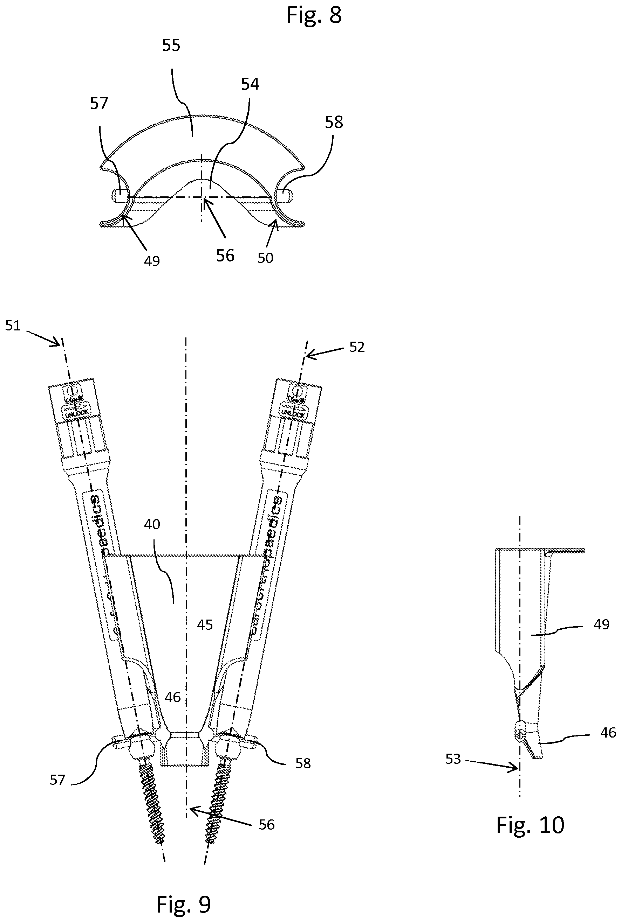

Les figures 8 à 10 représentent une alternative de réalisation où les gouttières (42, 43) viennent s'appuyer sur les surfaces extérieures(49, 50) des tubes (1, 2), de part et d'autrede la lumière longitudinale (16, 26) du tube (1, 2) associé. La zone médiane (40) forme un voile tronconique s'étendant d'une gouttière (42) à l'autre (43), pour créer une ouverture d'environ 20° entre les axes longitudinaux des deux gouttières (42, 43). Ce voile présente une courbure régulière, sans angles vifs susceptibles de blesser les tissus écartés. Lorsque l'accessoire est en place, il dégage un volume intérieur tronconique vide (54) constituant une zone de travail permettant l'introduction d'instruments chirurgicaux tels que des pinces à disques ou des pinces dites Kerisson (nom commercial) ou une cage intersomatique. La section d'ouverture de cette zone de travail tronconique présente un rayon d'environ 40 millimètres, et se restreint pour atteindre un rayon d'environ 15 millimètres à l'extrémité distale. La longueur de cette zone de travail est comprise entre c et 100 millimètres.

Une collerette (55) s'étend dans un plan sensiblement perpendiculaire à l'axe médian (56) de l'accessoire, sur une largeur d'environ 10 millimètres. Cette collerette (55) assure le renforcement de la pièce, et facilite sa manipulation.

A l'extrémité proximale, opposée à la dite collerette (55), le voile (41) présente un épanouissementcylindrique évasé (46).

Chacune des gouttière (42, 43) présente à sa surface d'appui sur le tube (1, 2) un ergot (57, 58) d'une longueur d'environ 10 millimètres et d'une section correspondant à l'ouverture de la lumière (16, 26) des tubes (1, 2) et au logement en U prévu dans la tête des vis rachidiennes (7, 8). Ces ergots viennent se loger dans les têtes de vis lorsque l'accessoire est repoussé en direction proximale. Ils assurent ainsi le verrouillage et l'immobilisation de l'ensemble par l'intermédiaire de l'insert ou d'un bouchon introduit dans le tube (1, 2).

### Description d'un exemple de mise en oeuvre

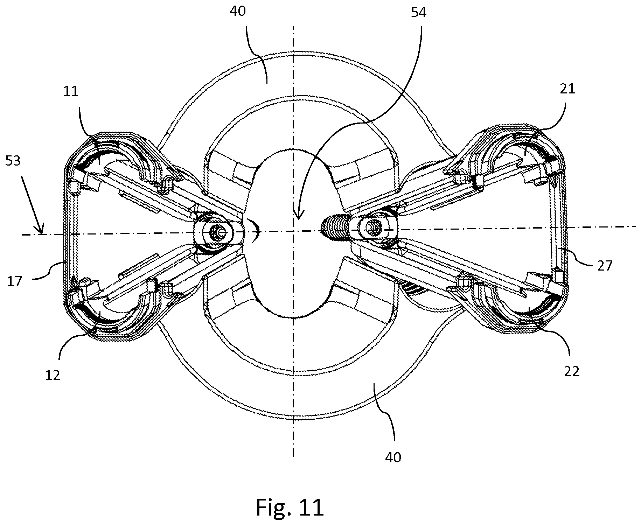

La figure 11 représente un exemple de mise en oeuvre d'un système utilisant les deux types d'accessoires.

Les tubes (1, 2) sont écartés par rapport à un plan longitudinal (53) en position ouvertes, les demi-coques (11, 12 ; 21, 22) étant verrouillés par des premiers accessoires (17, 27).

Ils sont également écartés par rapport un plan sagittal (59), perpendiculaire au plan longitudinal (53), à l'aide des seconds accessoires (40).

L'ensemble définit une zone de travail tronconique (54).

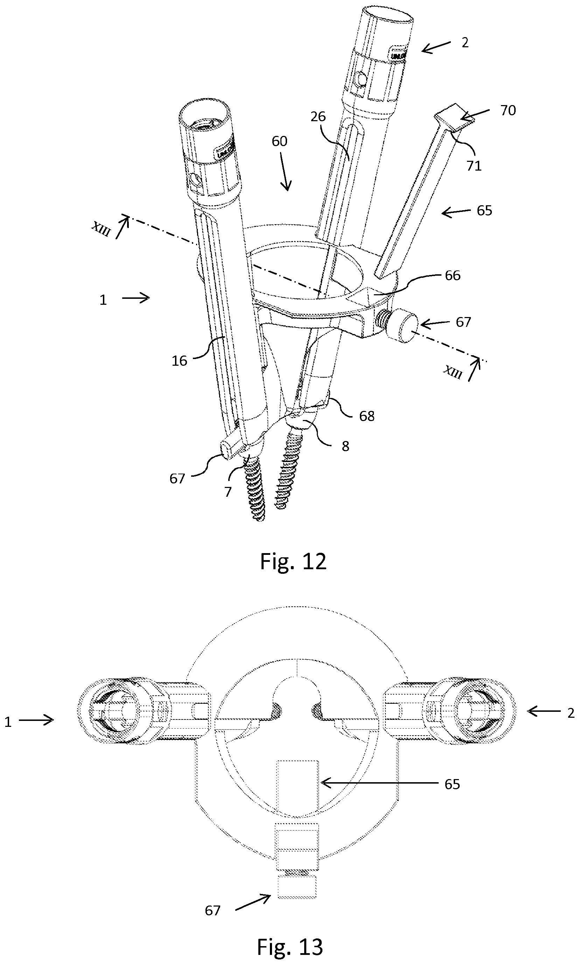

La figure 12 représente une vue en perspective d'une troisième variante de réalisation de deux tubes reliés par un troisième accessoire de liaison.

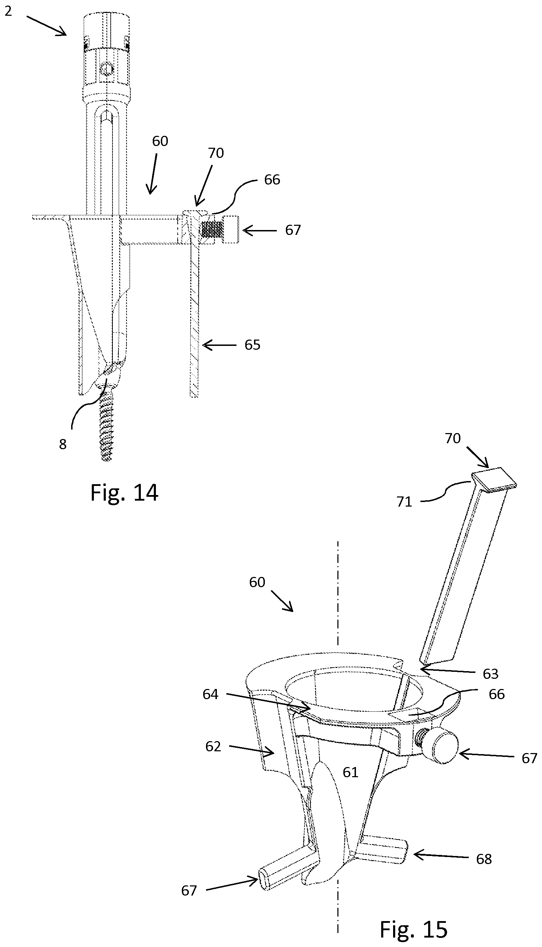

Le troisième accessoire de liaison 60, illustré sur la figure 15, reprend l'essentiel des caractéristiques de l'accessoire de liaison illustré sur la figure 9. En particulier, il comporte une zone médiane (61) présentant la forme d'un segment tronconique mince pour former un voile bombé. La zone médiane (61) est prolongée de part et d'autre par une gouttière (62, 63) de forme complémentaire à la section extérieure des tubes (1, 2). Plus particulièrement, les gouttières (62, 63) sont disposées diamétralement opposées l'une de l'autre. Elles sont en outre agencées pour venir s'appuyer sur la surface extérieure des tubes (1, 2), de part et d'autre de la lumière longitudinale (16, 26) du tube associé (1, 2) comme illustré sur la figure 12. De même, le troisième accessoire de liaison (60) comporte, en extrémité proximale, deux ergots (67, 68) s'étendant de part et d'autre de la zone médiane (61). Chaque ergots (67, 68) présente une section correspondant à l'ouverture de lumière (16, 26) des tubes (1, 2) et au logement en U prévu dans la tête des vis rachidiennes (7, 8). Comme indiqué précédemment, de tels ergots permettent d'assurer le verrouillage et l'immobilisation de l'ensemble tube et vis rachidienne lorsque l'accessoire de liaison (60) est positionné sur les tubes (1, 2), les ergots (7, 8) étant disposés dans la forme en U des têtes de vis (7, 8).

Dans cette variante de réalisation cependant, la zone médiane (61) est prolongée, en extrémité distale, par une couronne semi circulaire (64) s'étendant d'une gouttière (62) à l'autre gouttière (63). La couronne semi-circulaire (64) s'étend dans un plan sensiblement perpendiculaire à l'axe médian (56) de l'accessoire. Elle délimite, avec l'extrémité distale de la zone médiane (61) une ouverture d'entrée vers une zone de travail pour le passage d'instruments chirurgicaux ou d'implants. Dans le mode de réalisation illustré, la couronne semi-circulaire (64) est formée d'un seul tenant avec la zone médiane (61).

La couronne semi-circulaire (64) comporte un trou traversant (66) pour le passage d'une lame amovible (65) depuis l'extrémité distale de l'accessoire vers l'extrémité proximale. Avantageusement, le trou traversant (66) est placé de manière équidistante aux deux gouttières (62, 63). Lorsqu'elle est montée sur la couronne, la lame amovible (65) délimite avec le voile tronconique la zone de travail. Comme on le comprendra plus loin, la lame amovible (65) constitue une lame articulée.

Dans le mode de réalisation illustré, la lame amovible (65) est bloquée sur la couronne semi-circulaire (64) à l'aide d'une vis de blocage (67) montée dans un trou de passage ménagé sur la face extérieure de la couronne semi-cylindrique et débouchant dans le trou de passage (66) de la lame amovible (65). Il est bien entendu évident qu'il peut être prévu d'autres moyens de blocage de la lame sans sortir du cadre de l'invention.

Comme représenté sur la figure 14, le trou de passage (66) présente une forme en entonnoir se rétrécissant dans une direction opposée à l'extrémité distale de l'accessoire 60. Il présente en outre, en sortie, une section, légèrement supérieure à la section de la lame amovible (65). Cette configuration permet ainsi non seulement de faciliter l'insertion de la lame (65) dans le trou (66) mais également d'élargir la zone de travail par basculement de ladite lame dans le trou de passage (66) depuis une position dans laquelle la lame amovible converge vers l'extrémité proximale du voile tronconique vers une position sensiblement verticale (figure 14).

Avantageusement, la lame amovible (65) comporte en extrémité distale un moyen de préhension 70. Dans l'exemple illustré, le moyen de préhension est représenté par une forme en T (71).

## Revendications

1. - Dispositif rachidien pour la réalisation d'une intervention chirurgicale sur des vertèbres par voie postérieure ou postéro-latérale, comprenant
un ensemble constitué de deux éléments, chaque élément de l'ensemble étant une demi-coque ou un tube présentant respectivement une extrémité dite proximale apte à coopérer avec une vis rachidienne et une extrémité dite distale, et
un accessoire de liaison (17, 27, 40, 60) comprenant une partie médiane (30, 41, 61) pourvue de part et d'autre par une zone d'accrochage réversible sur chacun des éléments de l'ensemble,
**caractérisé en ce que** les zones d'accrochage sont arrangées avec la partie médiane (30, 41, 61) pour maintenir les éléments de l'ensemble l'un par rapport à l'autre dans une position angulaire ouverte telle que chaque élément de l'ensemble converge l'un vers l'autre en direction proximale.

2. - Dispositif rachidien selon la revendication 1, **caractérisé en ce que** les zones d'accrochage comprennent des parties arquées définissant des gouttières d'accrochage dont les axes convergent l'un vers l'autre en direction proximale.

3. - Dispositif rachidien selon la revendication 1 ou la revendication 2, **caractérisé en ce que** les tubes(1, 2) constituant les éléments de l'ensemble sont formés chacun de deux demi-coques (11, 12 ; 21, 22) aptes à coopérer avec la tête d'une vis rachidienne d'une manière permettant un basculement proximal de l'une au moins desdites demi-coques (11, 12, 21, 22) par rapport à la tête de ladite vis rachidienne (5, 6), ledit accessoire de liaison (17, 27, 40) étant configuré pour maintenir deux desdites demi-coques (11, 12, 21, 22) desdits tubes avec une orientation angulaire ouverte.

4. - Dispositif rachidien selon l'une quelconque des revendications 1 à 3, **caractérisé en ce que** ledit accessoire de liaison (17, 27) est constitué par une pièce adaptable auxdits éléments de l'ensemble, ladite pièce (17, 27) présentant une partie médiane (30) prolongée de part et d'autre par une zone d'accrochage réversible (31, 32) apte à coopérer avec une zone distale complémentaire d'une desdites demi-coque, afin de verrouiller lesdites demi-coques en position ouverte.

5. - Dispositif rachidien selon l'une quelconque des revendications 1 à 4, caractérisé en ce les zones d'accrochage (31, 32) présentent une surface intérieure complémentaire de la surface extérieure de la zone frontale des éléments de l'ensemble sur laquelle les zones d'accrochage s'accrochent.

6. - Dispositif rachidien selon l'une quelconque des revendications 1 à 5, **caractérisé en ce que**, lorsque les éléments de l'ensemble sont des tubes, ledit accessoire de liaison est constitué par une lame de rétraction (40) rigide apte à coopérer avec lesdits tubes (1, 2) distincts et à maintenir écartés les tissus de l'abord chirurgical par un verrouillage de l'orientation relative desdits tubes (1, 2).

7. - Dispositif rachidien selon la revendication 6, **caractérisé en ce que** la partie médiane (41) de la lame de rétractation (40) présente la forme d'un segment tronconique mince, pour former un voile bombé, prolongé de part et d'autre par une gouttière (42, 43) de forme complémentaire à la section extérieure desdits tubes, lesdites gouttières (42, 43), formant les zones d'accrochage réversible de l'accessoire de liaison.

8. - Dispositif rachidien selon la revendication 7, **caractérisé en ce que** les gouttières sont arrangées pour autoriser un déplacement relatif longitudinal des tubes (1, 2) logés dans lesdites gouttières (42, 43).

9. - Dispositif rachidien selon l'une quelconque des revendications 1 à 8, **caractérisé en ce que**, lorsque les éléments de l'ensemble sont des tubes, l'accessoire de liaison (40, 60) comporte, en extrémité proximale, des ergots (57, 58, 67, 68) assurant le verrouillage et l'immobilisation des tubes sur les vis rachidienne lorsque ces derniers sont montés sur les vis rachidiennes (7, 8) et lorsque l'accessoire de liaison (60) est positionné sur les tubes (1, 2), lesdits ergots (57, 58, 67, 68) s'étendant de part et d'autre de la zone médiane (41, 61) et présentant une section correspondant au logement en U de la tête de vis rachidienne (7, 8).

10. - Dispositif rachidien selon l'une au moins des revendications 1 à 9, **caractérisé en ce qu'**il comprend deux tubes (1, 2) constitués chacun de deux demi-coques (11,12, 21,22), de deux vis rachidiennes (5, 6) et deux accessoires de liaison.

11. - Dispositif rachidien selon la revendication 7 ou la revendication 8, **caractérisé en ce que** la zone médiane (41) présente une première partie distale (45) en forme de tuile tronconique de section décroissante vers la direction proximale, prolongée par une deuxième partie proximale (46) en forme de tuile cylindrique, évasée par rapport à la première partie distale (45).

## Patentansprüche

1. Vorrichtung für den chirurgischen Eingriff an Wirbeln auf dem posterioren oder posterior-lateralen Weg, wobei die Vorrichtung folgendes umfasst:
eine Einheit bestehend aus zwei Elementen, wobei jedes Element der Einheit eine Halbschale oder eine Röhre ist, die jeweils ein sogenanntes proximales Ende aufweist, welches in der Lage ist, mit einer Wirbelschraube und einem sogenannten distalen Ende zusammen zu wirken, und
ein Verbindungszubehörteil (17, 27, 40, 60), welches einen mittleren Teil (30, 41, 61) umfasst, der zu beiden Seiten durch einen Bereich für die reversible Befestigung auf jedem der Elemente der Einheit versehen ist, **dadurch gekennzeichnet, dass** die Befestigungsbereiche so mit dem mittleren Teil (30, 41, 61) angeordnet sind, dass sie die Elemente der Einheit zueinander in einer offenen Winkelposition halten, so dass jedes Element der Einheit das andere in proximale Richtung konvergiert.

2. Vorrichtung für den Eingriff an der Wirbelsäule nach Anspruch 1, **dadurch gekennzeichnet, dass** die Befestigungsbereiche bogenförmige Partien umfassen, welche die Befestigungsschienen, deren Achsen in proximale Richtung zueinander konvergieren, abgrenzen.

3. Vorrichtung für den Eingriff an der Wirbelsäule nach Anspruch 1 oder Anspruch 2, **dadurch gekennzeichnet, dass** die Röhren (1, 2), welche die Elemente der Einheit bilden, jeweils durch zwei Halbschalen (11, 12; 21, 22) gebildet werden, die in der Lage sind, mit dem Kopf einer Wirbelschraube zusammen zu wirken, so dass das proximale Kippen mindestens einer der Halbschalen (11, 12, 21,22) in Bezug auf den Kopf besagter Wirbelschraube (5, 6) möglich ist, wobei besagtes Verbindungzubehörteil (17, 27, 40) so gestaltet ist, dass zwei der besagten Halbschalen (11, 12, 21, 22) besagter Röhren in einer offenen winkelförmigen Ausrichtung gehalten werden.

4. Vorrichtung für den Eingriff an der Wirbelsäule gemäß einem der Ansprüche 1 bis 3, **dadurch gekennzeichnet, dass** besagtes Verbindungszubehörteil (17, 27) aus einem Teil besteht, das an besagte Elemente der Einheit angepasst werden kann, wobei besagtes Teil (17, 27) einen mittleren Teil (30) aufweist, der zu beiden Seiten durch einen Bereich für eine reversible Befestigung (31, 32) verlängert wird, welcher in der Lage ist, mit einem distalen ergänzenden Bereich einer der besagten Halbschalen zusammen zu wirken, um besagte Halbschalen in offener Position zu verriegeln.

5. Vorrichtung für den Eingriff an der Wirbelsäule gemäß einem der Ansprüche 1 bis 4, **dadurch gekennzeichnet, dass** die Befestigungsbereiche (31, 32) eine Innenfläche aufweisen, welche die Außenfläche des vorderen Bereichs der Elemente der Einheit, auf welcher die Befestigungsbereiche befestigt werden, ergänzt.

6. Vorrichtung für den Eingriff an der Wirbelsäule nach einem der Ansprüche 1 bis 5, **dadurch gekennzeichnet, dass** wenn die Elemente der Einheit Röhren sind, besagtes Verbindungszubehörteil aus einer steifen Einzugsfeder (40) besteht, die in der Lage ist, mit besagten einzelnen Röhren (1, 2) zusammen zu wirken und das Gewebe im Operationsbereich durch die Verriegelung der Ausrichtung in Bezug auf besagte Röhren (1, 2) gespreizt zu halten.

7. Vorrichtung für den Eingriff an der Wirbelsäule nach Anspruch 6, **dadurch gekennzeichnet, dass** der mittlere Teil (41) der Einzugsfeder (40) die Form eines dünnen konusförmigen Abschnitts hat, um einen gewölbten Beschlag zu bilden, der zu beiden Seiten durch eine Schiene (42,43) in einer den äußeren Abschnitt besagter Röhre ergänzenden Form verlängert wird, wobei besagte Schienen (42, 43) die Bereiche der reversiblen Befestigung des Verbindungszubehörteils bilden.

8. Vorrichtung für den Eingriff an der Wirbelsäule nach Anspruch 7, **dadurch gekennzeichnet, dass** die Schienen so angeordnet sind, dass sie eine relative Längsverschiebung der in besagten Schienen (42,43) liegenden Röhren (1, 2) ermöglichen.

9. Vorrichtung für den Eingriff an der Wirbelsäule nach einem der Ansprüche 1 bis 8, **dadurch gekennzeichnet, dass** wenn die Elemente der Einheit Röhren sind, das Verbindungszubehörteil (40, 60) am proximalen Ende Stifte (57, 58, 67, 68) aufweist, welche die Verriegelung und das Blockieren der Röhren auf den Wirbelschrauben gewährleisten, wenn letztere auf die Wirbelschrauben (7, 8) montiert werden und wenn das Verbindungszubehörteil (60) auf den Röhren (1, 2) positioniert wird, wobei sich besagte Stifte (57, 58, 67, 68) zu beiden Seiten des mittleren Bereichs (41, 61) erstrecken und einen Abschnitt aufweisen, der der u-förmigen Aufnahme des Kopfes der Wirbelschraube (7, 8) entspricht.

10. Vorrichtung für den Eingriff an der Wirbelsäule nach mindestens einen der Ansprüche 1 bis 9, **dadurch gekennzeichnet, dass** sie zwei Röhren (1, 2) umfasst, welche jeweils aus zwei Halbschalen (11, 12, 21, 22), zwei Wirbelschrauben (5, 6) und zwei Verbindungszubehörteilen gebildet werden.

11. Vorrichtung für den Eingriff an der Wirbelsäule nach Anspruch 7 oder Anspruch 8, **dadurch gekennzeichnet, dass** der mittlere Bereich (41) einen ersten distalen Teil (45) in Form eines konusförmigen Formteils aufweist, deren Querschnitt in proximale Richtung kleiner wird, die durch einen zweiten proximalen Teil (46) in Form eines zylindrischen Formteils, welches in Bezug auf den ersten distalen Teil (45) ausgestellt ist, verlängert wird.

## Claims

1. Rachidian device for implementing surgery on vertebrae by posterior or posterolateral method, comprising
an assembly consisting of two elements, each element of the assembly being a half-shell or a tube having respectively a so-called proximal end able to cooperate with a rachidian screw and a distal end, and
a connecting accessory (17, 27, 40, 60) comprising a middle part (30, 41, 61) provided on either side with a zone for reversible attachment on each of the element of the assembly,
**characterised in that** the attachment zones are arranged with the middle part (30, 41, 61) so as to hold the elements of the assembly with respect to one another in an open angular position such that each element of the assembly converges towards the other in the proximal direction.

2. Rachidian device according to claim 1, **characterised in that** the attachment zones comprise arched parts defining attachment channels, the axes of which converge towards each other in the proximal direction.

3. Rachidian device according to claim 1 or claim 2, **characterised in that** the tubes (1, 2) constituting the elements of the assembly are each formed by two half-shells (11, 12; 21, 22) able to cooperate with the head of a rachidian screw in a way allowing proximal tilting of at least one of said half-shells (11, 12; 21, 22) with respect to the head of said rachidian screw (5, 6), said connecting accessory (17, 27, 40) being configured so as to hold said half-shells (11, 12; 21, 22) of said tubes with an open angular orientation.

4. Rachidian device according to any of claims 1 to 3, **characterised in that** said connecting accessory (17, 27) consists of a part adaptable to said elements of the assembly, said part (17, 27) having a middle part (30) extended on either side by a reversible attachment zone (31, 32) able to cooperate with a complementary distal zone of one of said half-shells, in order to lock said half-shells in the open position.

5. Rachidian device according to any of claims 1 to 4, **characterised in that** the attachment zones (31, 32) have an internal surface complementary to the external surface of the frontal zone of the elements of the assembly to which the attachments zones are attached.

6. Rachidian device according to any of claims 1 to 5, **characterised in that**, when the elements of the assembly are tubes, said connecting accessory is formed by a rigid retraction blade (40) able to cooperate with said separate tubes (1, 2) and to keep the tissues of the surgery area separated by locking the relative orientations of said tubes (1. 2).

7. Rachidian device according to claim 6, **characterised in that** the middle part (41) of the retraction blade (40) is in the form of a thin frustoconical segment, in order to form a curved web, extended on either side by a channel (42, 43) with a shape complementary to the external cross section of said tubes, said channels (42, 43) forming the reversible attachment zones of the connecting assembly.

8. Rachidian device according to claim 7, **characterised in that** the channels are arranged so as to allow the longitudinal relative movement of the tubes (1, 2) housed in said channels (42, 43).

9. Rachidian device according to any of claims 1 to 8, **characterised in that**, when the elements of the assembly are tubes, the connecting accessory (40, 60) comprises, at the proximal end, lugs (57, 58, 67, 68) providing the locking and immobilising of the tubes on the rachidian screws (7, 8) and when the connecting accessory (60) is positioned on the tubes (1,2), said lugs (57, 58, 67, 68) extending on either side of the middle zone (41, 61) and having a cross section corresponding to the U-shaped housing of the head of the rachidian screw (7,8).

10. Rachidian device according to at least one of claims 1 to 9, **characterised in that** it comprises two tubes (1, 2) each consisting of two half-shells (11, 12, 21, 22), two rachidian screws (5, 6) and two connecting accessories.

11. Rachidian device according to claim 7 or claim 8, **characterised in that** the middle zone (41) has a first distal part (45) in the form of a frustoconical tile with a cross section decreasing towards the proximal direction, extended by a second proximal part (46) in the form of a cylindrical tile, splayed with respect to the first distal part.
